# EUROPEAN PATENT SPECIFICATION

(11) **EP 0 650 350 B1**
(45) Date of publication and mention of the grant of the patent: **10.06.1998**
(21) Application number: 93918350.5
(22) Date of filing: 22.07.1993
(51) Int. Cl.: A61F 13/74

(54) **MENSTRUAL SHORTS WITH IMPROVED PERINEUM CONTACT**
MONATSHÖSCHEN MIT VERBESSERTEM PERINEALEN KONTAKT
CULOTTES PERIODIQUES AYANT UN CONTACT AMELIORE AVEC LE PERIMEE

(30) Priority: 23.07.1992 US 915133
(43) Date of publication of application: 03.05.1995
(73) Proprietor: THE PROCTER & GAMBLE COMPANY, Cincinnati, Ohio 45202 (US)
(72) Inventor: OSBORN, Thomas, Ward, III, Cincinnati, OH 45224 (US); SCHMITZ, Deborah, Catherine, West Chester, OH 45069 (US); REDWINE, Nona, Jane, Mason, OH 45040 (US)
(74) Representative: Hirsch, Uwe Thomas
(86) International application number: PCT/US93/06947
(87) International publication number: WO 94/02099

(56) References cited:
- EP-A- 0 335 253
- EP-A- 0 357 298
- WO-A-90/04374
- DE-A- 435 579
- US-A- 2 052 598
- US-A- 3 608 551

## Description

### BACKGROUND OF THE INVENTION

### 1. Technical Field

This invention relates to feminine menstrual protection devices and, more particularly, to menstrual shorts incorporating a lifting mechanism for positioning a catamenial pad in the pudendal region of the female wearer.

### 2. Background Art

As is known, disposable catamenial pads are commercially available in a wide variety of configurations for the specific purpose of absorbing and retaining menstrual fluids and other vaginal discharges. Regardless of the specific configuration, all catamenial pads are intended to isolate the discharged menses for preventing the undergarments, clothing, bedding etc. surrounding the disposable article from becoming soiled. Unfortunately, a primary failure mechanism associated with catamenial pads is leakage of menses from a rear portion thereof due to poor fit in the gluteal groove and against the perineum of the female wearer.

To provide additional protection against leakage, it is known to use a washable and reusable garment, such as a menstrual short or panty, in combination with a disposable catamenial pad. For example, U.S. Patent 3,489,149 to Larson discloses a washable menstrual panty having a small pocket in the crotch area for retaining a disposable catamenial pad. Since the menses must initially flow through a layer of material forming the pocket to reach the catamenial pad, removal of the soiled catamenial pad can be distasteful, difficult and unsanitary. While a new pad can be inserted into the pocket, the garment is already soiled and would typically be changed. Also, the pocket may not accommodate the varied sizes of catamenial pads currently on the market. Finally, the menstrual panty is not adapted to deliberately position the catamenial pad for preventing leakage of menses from the rear portion thereof.

Various other menstrual shorts or panties are known which also fail to adequately address the need for properly positioning and retaining the disposable catamenial pad in the pudendal region of the female wearer. For example, U.S. Patent 4,560,381 to Southwell describes a mesh-like outer panty shell with a thick inner layer of absorbent material in the lower crotch area of the panty. The inner layer of absorbent material includes a depression for receiving and positioning a catamenial pad. In U.S. Patent 4,813,950 to Branch, a washable menstrual panty is disclosed as having an outer lining of spandex, soft tricot, etc. and an inner lining of soft plastic film to prevent passage of menses therethrough. US 3 608 551 discloses menstrual short panties with an integrally formed elastic tape located on the top of the crotch portion. Similarly, US 2 052 598 discloses a sanitary garment having an elastic panel secured over the crotch portion. Alternatively, existing Japanese-style menstrual shorts act like a girdle or very tight fitting panty which holds the catamenial pad in the wearer's pudendal region. However, positioning of the catamenial pad is achieved by the tightness of the menstrual short which, in turn, may cause discomfort to the wearer.

In each of the above-noted menstrual shorts, the catamenial pad is not positioned and retained by the menstrual shorts in the gluteal groove and against the perineum to prevent leakage of menses from the rear portion of the catamenial pad. Rather, such known menstrual garments are typically designed to position the catamenial pad near the vaginal vestibule and attempt to prevent leakage of menses by using liquid impervious layers or boundaries.

It is therefore an object of the present invention to provide an improved menstrual garment having a lifting mechanism for locating and retaining a disposable catamenial pad in the gluteal groove and against the perineum of the female wearer's body. Labial contact may also be provided.

It is a related object of the present invention to provide a menstrual short or panty that maintains enhanced pudendal/pad contact for reducing leakage of menses from the rear of the catamenial pad, and yet may be looser fitting and more comfortable than conventional menstrual garments.

These and various other objectives of the present invention will be more readily apparent when considered in reference to the following description and when taken in conjunction with the accompanying drawings.

### SUMMARY OF THE INVENTION

The menstrual garment of the present invention is a washable and reusable panty or short having an outer layer defining first and second leg openings for receiving the legs of a female wearer. In addition, an inner layer or gusset-type liner is provided between the first and second leg openings and is attached to the outer layer. A disposable catamenial pad is retained on the inner layer. To provide means for lifting the disposable catamenial pad into the gluteal groove and against the perineum of the wearer, the menstrual short includes a lifting mechanism. In one preferred form, the lifting mechanism is an elongated cinch that is located between the inner and outer layers. The cinch is adapted to lift the inner layer which, in turn, lifts the catamenial pad into the gluteal groove and against the perineum of the female, thereby providing sustained body contact for inhibiting leakage of menses from a rear portion of the catamenial pad. In addition, the menstrual short may further include a guide for maintaining alignment of the cinch along a center portion of the inner layer. Alternatively, the lifting mechanism can include a stitched ridge of seam that is sewn into the inner layer for lifting the catamenial pad in a similar manner.

### BRIEF DESCRIPTION OF THE DRAWINGS

While the specification concludes with claims particularly pointing out and distinctly claiming the subject matter which is regarded as forming the present invention, it is believed that the present invention will be better understood from the following description when taken in conjunction with the accompanying drawings in which:
Figure 1 is a front view of a menstrual short according to the prior art and which is shown being worn by a female;
Figure 2 is a front view of an improved menstrual short incorporating a cinch-type lifting mechanism according to the present invention;
Figure 3 is plan view of the menstrual short of Figure 2;
Figure 4 is an enlarged plan view of a portion of the menstrual short shown in Figure 3;
Figure 5 is a side view of the menstrual short of Figure 2;
Figure 6 is a rear view of the menstrual short of Figure 2 folded flat along lateral sides thereof;
Figure 7 is a plan view of an alternate embodiment for the lifting mechanism of the present invention showing a gusset-type liner incorporating a stitched seam;
Figure 8 is a side view of the alternate embodiment of Figure 7;
Figure 9 is an enlarged plan view of a portion of a menstrual short incorporating a deformation element according to a third embodiment of the present invention;
Figure 10A is an end view of a first deformation element shown in a relaxed state;
Figure 10B is an end view of the first deformation element of Figure 10A illustrating lateral forces applied along lateral edges of the first deformation element;
Figure 11 A is an end view of a second deformation element according to third embodiment of the present invention in a relaxed state; and
Figure 11B is an end view of the second deformation element of Figure 11A with lateral forces applied along lateral edges thereof.

### DESCRIPTION OF THE PREFERRED EMBODIMENT

The present invention relates generally to washable and reusable menstrual garments and, more particularly, to a menstrual short or panty incorporating a lifting mechanism for lifting a disposable catamenial pad into enhanced contact with the wearers pudendal region. As an additional feature, the lifting mechanism is adapted to cause the catamenial pad to be lifted into intimate contact with the gluteal groove and against the perineum of the female wearer for inhibiting leakage of menses for the rear portion of the catamenial pad. Enhanced labial contact may be provided as well.

As used herein, the term "disposable catamenial pad" refers to an absorbent article which is worn by females adjacent to the pudendal region for absorbing and containing menstrual fluids and other vaginal discharges, and which is intended to be discarded after a single use. As used herein, the term "pudendal" refers to the externally visible female genitalia and is limited to the labia majora, the labia minora, the clitoris and the vaginal vestibule. In addition, the term "perineum" refers to the external region of the female's body between the anus and the pudendal region while the term "gluteal groove" refers to the separation between the buttocks extending upwardly from the perineum.

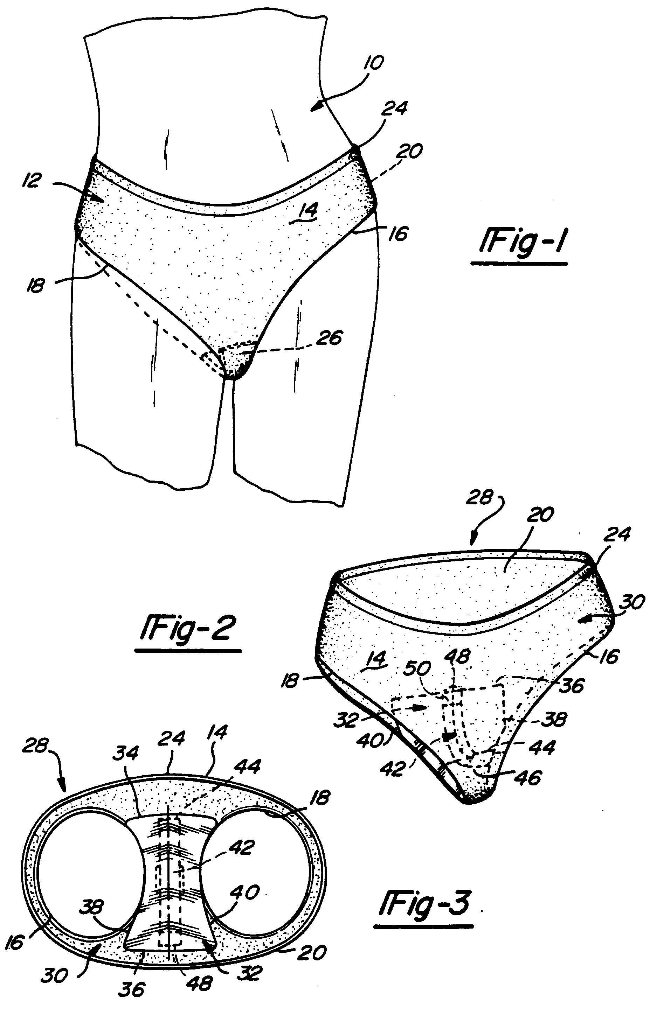

In Figure 1, a female 10 is shown wearing a prior art menstrual short or panty 12 having a front portion 14, first and second leg openings 16 and 18, a rear portion 20, and an elastic waistband 24 for maintaining the menstrual short 12 on the female 10. The panty 12 can be any style, for example "bikini", "tanga", "French cut", American style, or Japanese menstrual shorts. A disposable catamenial pad 26 is shown positioned by the menstrual short 12 adjacent the perineum and pudendal regions of the female 10 in a conventional manner.

Referring now to Figures 2 through 5, a menstrual short or panty 28 incorporating the novel features of the present invention is shown. For purposes of clarity, like reference numerals are used where appropriate. In general, menstrual short 28 is an improvement over prior art menstrual short 12 in view of the incorporation of a lifting mechanism into menstrual short 28 which is adapted to lift the catamenial pad 26 into enhanced contact with the female's pudendal and perineum regions and in the gluteal groove to provide sustained placement relative thereto under all motion and use conditions. As a result, the likelihood of leakage of menses from the rear of the catamenial pad 26 (i.e., along the perineum or the gluteal groove) is significantly reduced.

The menstrual short 28 includes an outer layer 30 defining the "shell" of the panty worn by the female wearer and an inner protective layer or gusset-type liner 32. As seen, outer layer 30 includes a front portion 14, a rear portion 20, leg openings 16 and 18, and an elastic waistband 24. The gusset-type liner 32 is attached to the outer layer 30 in the crotch area of the menstrual short 28 and extends from a region anterior to the vaginal vestibule of the female 10 toward the elastic waistband 24 in the rear portion 20 of outer layer 30. More particularly, the gusset 32 has a generally hour-glass shape and includes a front edge 34, a rear edge 36, and a pair of lateral edges 38 and 40.

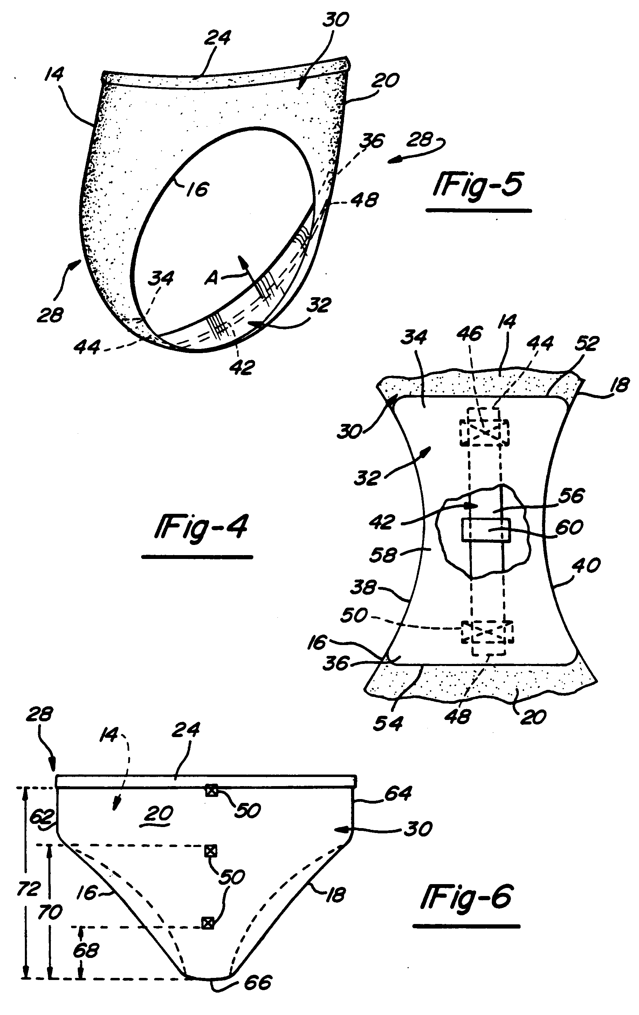

In accordance with the present invention, a lifting mechanism is provided between the gusset-type liner 32 and the outer layer 30 for lifting gusset 32 toward the perineum and gluteal groove while providing some degree of inter labial contact. More particularly, the lifting mechanism provides upward lift to the gusset 32 which, in turn, lifts the disposable catamenial pad 26 worn by the female 10 into her gluteal groove and against her perineum. As a result, leakage of menses from a rear portion of the catamenial pad 26 is avoided, thereby inhibiting the undesirable soiling of the menstrual short 28 and other surrounding clothing or bedding. In general, this "lifting" action is obtained by using the differential stretch attributes of the various components of menstrual short 28 and/or the specific attachment locations for these components. In one preferred form, the lifting mechanism is a ridge or cinch 42 that is shown in Figure 4 lifting the gusset-type liner 32 in an upward direction, as indicated by arrow "A". The cinch 42 is an elongate strip of an elastic material. The cinch 42 should be made of an elastic material requiring less that 800 grams of force to elongate 25%. A more preferred material requires less than 400 grams of force. A highly preferred material requires less than 200 grams of force. To obtain the above measurements, the cinch material is mounted in a elongation device for example an INSTRON™. The cinch material is pulled until it breaks or tears using a gage length of 2.00" and a strain rate of 10"/minute. The above force measurements ate taken at a 25% point on a force-elongation curve. Regardless of the specific material chosen, the cinch 42 should only provide sufficient upward lift to promote enhanced and sustained pad contact while preventing discomfort to the female 10 when worn.

With continued reference to Figures 2 through 6, a first or front end 44 of the cinch 42 is shown attached at a front attachment location 46 to either the outer layer 30 or gusset-type liner 32 so as to be located within the front portion 14 of the menstrual short 28. Similarly, a second or rear end 48 of the cinch 42 is shown attached at a rear attachment location 50 to either the gusset-type liner 32 or the outer layer 30 in the rear portion 20 of the menstrual short 28. Preferably, the front and rear ends 44 and 48 of the cinch 42 are fixedly attached (i.e., sewn, adhesively bonded, etc.) at the front and rear attachment locations 46 and 50, respectively. Specific preferred attachment locations for the front and rear ends 44 and 48 of the cinch 42 will be described in greater detail below.

To achieve the requisite upward lift of gusset-type liner 32, the front attachment location 46 of the cinch 42 should be located at least anterior to the anus of the female 10 when worn. More preferably, the front attachment location 46 of the cinch 42 should be located adjacent the vaginal vestibule of the female 10 when worn. However, other front attachment locations 46 are readily apparent. Similarly, the rear attachment location 50 of the cinch 42 should be located sufficiently high in the rear portion 20 of the menstrual shorts 28 to provide the upward lift required to bias the disposable catamenial pad 26 into the gluteal groove and against the perineum of the female 10. Positioning the rear attachment location 50 above the curvature of the buttocks of the female wearer 10 is typically sufficient to provide the desired amount of vertical lift. Positioning the rear attachment location 50 in close proximity to the waistband 24 is also desirable. However, other rear attachment locations 50 are readily apparent. It will be appreciated that the specific position of the rear attachment location 50 relative to the waistband 24 will vary depending on the style of the menstrual short and comfort of the female 10 when the menstrual short 28 is worn. Furthermore, the shape of the buttocks of the female 10, which varies according to ethnic background; also effects the positioning of the rear attachment location 50 relative to the waistband 24.

From Figure 4, the opposite ends of cinch 42 are shown attached at front and rear attachment locations 46 and 48, respectively, to the outer layer 30. As noted, the cinch 42 can be attached to outer layer 30 in any suitable manner (i.e., sewn, adhesively bonded, hook and loop fasteners, etc.). As can also be seen, the front and rear edges 34 and 36 of the gusset 32 are attached at 52 and 54 to the outer layer 30. In this example, cinch 42 is completely disposed and retained between gusset-type liner 32 and outer layer 30. Alternatively, the cinch 42 can initially be attached to the gusset 32 and then the gusset 32 and cinch 42 can be attached as a sub-assembly to the outer layer 30. Other ways of attaching the gusset 32 and/or the cinch 42 to the outer layer 30 will be readily apparent to those skilled in the art. Similarly, the first and second lateral edges 38 and 40 of the gusset 32 can be attached to the outer protective layer in any suitable fashion, for example by sewing, adhesive bonding and the like.

As an additional feature of the present invention, means are provided for positioning an intermediate portion 56 of the cinch 42 along a central portion 58 (Figure 4) of the gusset 32. Preferably, a loop or guide 60 can be secured to one of gusset 32 or outer layer 30 for receiving and retaining intermediate portion 56 of cinch 42.

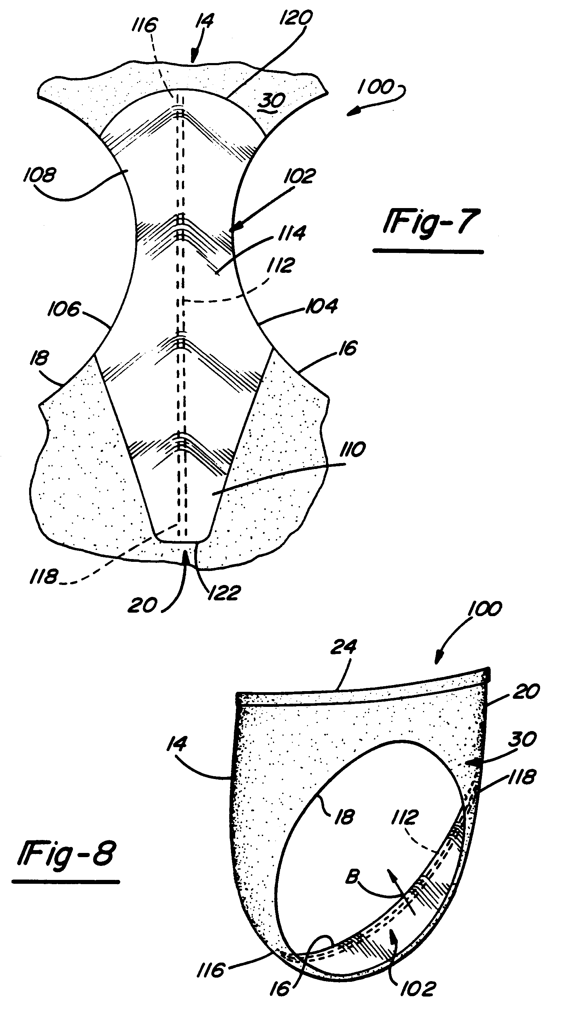

With particular reference to Figure 6, a rear view of the menstrual short 28 is shown folded flat along first and second lateral sides 62 and 64 thereof with various rear attachment locations 50 for the cinch 42 shown in greater detail. At a minimum, the rear attachment location 50 for the rear end 48 of the cinch 42 is preferably located at least 2.75 inches from a lowest portion 66 of the outer layer 30 when the menstrual short 28 is folded along the first and second sides 62 and 64, as shown at 68. However, the rear attachment location 50 is more preferably located at least six or seven inches from the lowest portion 66, as respectively shown at 70 and 72, when the menstrual short 28 is folded.

With reference to Figures 7 and 8, a second embodiment of the present invention is shown. More specifically, a menstrual short 100 is shown which includes a modified inner protective layer or gusset-type liner 102. The gusset-type liner 102 includes first and second lateral edges 104 and 106 which are attached adjacent to the leg openings 16 and 18 in any of the above-noted known methods. Front and rear portions 108 and 110 of the gusset-type liner 102 are respectively attached to the front portion 14 and the rear portion 20 of the menstrual short 100. An elongated stitched seam 112 is sewn into gusset-type liner 102 for lifting a center longitudinal portion 114 of gusset 102 in an upward direction, as indicated by arrow "B", and into the gluteal groove and against the perineum of the female 10. The front and rear ends 116 and 118 of stitched seam 112 need not extend fully to the distal ends 120 and 122 of the front and rear portions 108 and 110 of gusset-type liner 102. Functionally, the stitched seam 112 lifts the center longitudinal portion 114 of gusset 102 because the menstrual short 100 stretches from the front portion 14 to the rear 20 when the female 10 puts the menstrual short 100 on. The stitched seam 112 can be made of elastic thread or a non-elastic thread to obtain a desired amount of lift. Similarly, the width, length and pattern of the stitched seam 112 can be varied to provide the requisite lift. While seam 112 is shown formed in a surface of gusset 102 so as to be adjacent outer layer 30, it is contemplated that a stitched seam could alternatively be sewn into the exterior surface of gusset 102.

Location of the front and rear ends 116 and 118 of stitched seam 112 in relation to the front and rear portions 14 and 20 of the menstrual short 100 (and a length of the stitched seam 112) is analogous to the location of the front and rear attachment locations 46 and 50 of the cinch 42. In other words, the front end 116 of seam 112 should extend at least anterior to the anus of the female 10 when worn and the rear end 118 of seam 112 should extend at least 2.75" above the lowest portion of the outer layer 30 when the menstrual short 100 is folded flat along its first and second lateral sides 84 and 86.

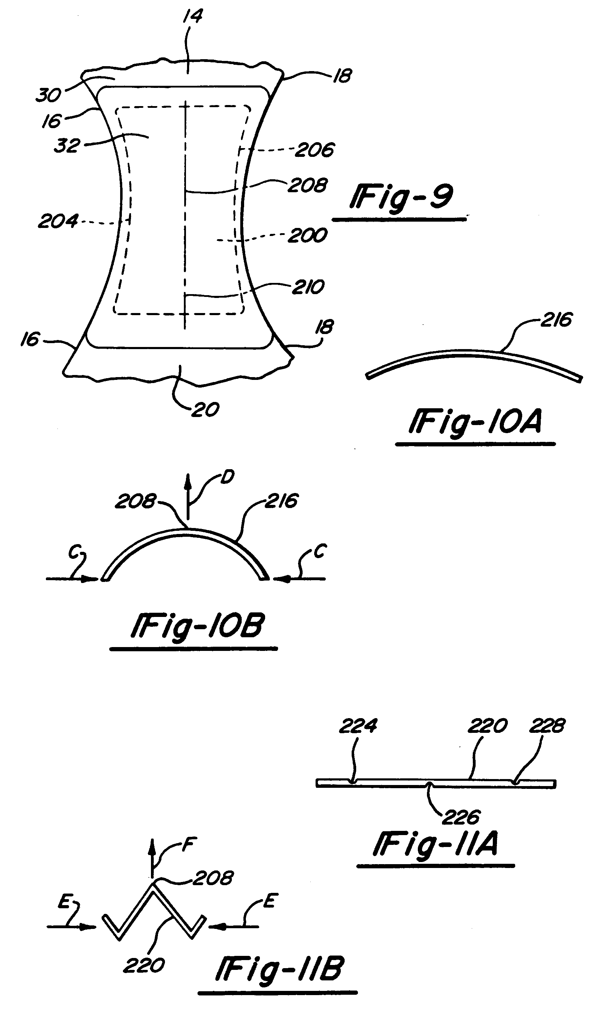

With reference to Figures 9-11, a third embodiment of the present invention incorporating a deformation element 200 as the lifting mechanism is illustrated. The thighs of the female wearer compress lateral outer edges 204 and 206 of the deformation element 200 causing a center portion 208 of the deformation element (indicated by dotted lines 210) to move in an upward direction similar to the direction indicated by the arrow "A" in Figure 4.

The deformation element 200 can be located between the inner protective layer 32 and the outer layer 30. The deformation element 200 may be affixed to an outer or body-facing surface of the inner protective layer 32. The deformation element may also be affixed to an outer surface of the outer protective layer 30.

The deformation element 200 should preferably extend between the first and second leg openings 16 and 18. If the panty or menstrual short 28 includes elastic around the leg openings 16, 18, the deformation element should extend and be attached just inside of the elastic. The deformation element should be greater than 2" in length. Preferably the deformation element has a length between 2.5" and 3.5".

In Figure 10, a first deformation element 216 having a "C"-shaped cross-section is shown. When the female wears the menstrual short 28, her thighs compress the deformation element inwardly as indicated by arrows "C" in Figure 10B. As a result, the center portion 208 moves in an upward direction indicated by arrow "D". As can be appreciated, the deformation element 216 biases the catamenial pad into the female's pudendal region, specifically into the gluteal groove and against the perineum. Labial contact may also be provided.

Referring to Figure 11, a second deformation element 220 has a straight cross-section when in a relaxed state and a "W"-shaped cross-section when in a compressed state. Compression lines 224, 226, and 228 formed parallel to a length of the second deformation element facilitate bending into the "W" shape under lateral compression forces of the female's thighs during use. When the female wears the menstrual short 28, her thighs laterally compress the deformation element inwardly as indicated by arrows "E" in Figure 11B. As a result, the center portion 208 moves in an upward direction indicated by arrow "F". As can appreciated, other shapes and sizes for the deformation element will be readily apparent.

The deformation element can be made of a variety materials such as thin plastic films, foams, or scrims. Polyethylene foam may be used. Alternatively, 30 mil polyethylene film may be used. Preferably the material used substantially returns to its original uncompressed shape when the lateral compression force is removed. Preferably the material used is laterally displaced at least 0.25" when 0.25 pounds are applied to the lateral edges 204 and 206.

As can be appreciated from the foregoing, menstrual shorts incorporating the present invention reduce soiling by providing enhanced body contact and sustained positioning of the catamenial pad under various body motions and user conditions. Additionally, the menstrual short or panty of the present invention may have a looser and more comfortable fit compared to prior art menstrual shorts while providing greater leakage prevention.

## Claims

1. A menstrual garment (28) for maintaining a catamenial pad (26) in a pudendal region of a female wearer comprising a washable and reusable panty or short (28) comprising:
an outer layer (30);
first (16) and second leg (18) openings formed in said outer layer (30) for receiving legs of the female wearer;
an inner layer (32) disposed between said first (16) and second leg (18) openings and attached to said outer layer (30) and said menstrual garment further comprising a catamenial pad retained on said outer layer (30) and said short (28) comprising
a lifting means selected from a cinch member (42), deformation member (200) or a stitched seam (112) characterised in that said lifting means is located between said inner layer (32) and said outer layer (30) for lifting said catamenial pad (26) when worn by the female wearer in the pudendal region into the gluteal groove and against the perineum of the female.

2. The menstrual garment of Claim 1 further comprising attachment means for securing said lifting means to one of said inner (32) and outer layers (32).

3. The menstrual garment of Claim 2 wherein said attachment means is a hook and loop fastening system.

4. The menstrual garment of Claim 2 wherein said attachment means is an adhesive.

5. The menstrual garment of Claim 1 wherein said lifting means is a clinch member (42).

6. The menstrual garment of Claim 5 wherein a front end of said cinch is attached to at least one of said inner and outer layers at a location that is anterior to the anus of the female.

7. The menstrual garment of Claim 5 wherein a front end of said cinch is attached to at least one of said inner and outer layers at a location that is adjacent the vaginal vestibule of the female.

8. The menstrual garment of Claim 5 further including locating means for positioning an intermediate portion of said cinch means along a center longitudinal portion of said inner layer.

9. The menstrual garment of Claim 5 wherein a rear end of said cinch is attached to at least one of said inner and outer layers at a location that is above the curvature of the buttocks of the female.

10. The menstrual garment of Claim 5 wherein a rear end of said cinch is attached to at least one of said inner and outer layers at a location that is adjacent an elastic waistband of said menstrual short.

11. The menstrual garment of Claim 5 wherein a rear end of said cinch is attached to at least one of said inner and outer layers in a position at least 2.75" from a lowest portion of said other layer when said menstrual short is folded flat along first and second lateral sides thereof.

12. The menstrual garment of Claim 5 wherein said cinch (42) is made of an elongated piece of elastic material having a first end (44) and a second end (48), said first end (44) being secured to one of said inner (32) and outer layers (30) at a location anterior to the anus of the wearer, and said second end (48) being secured to one of said inner (32) and outer layers (30) at a location that is adjacent the buttocks of the wearer.

13. The menstrual garment of Claim 12 further comprising guide means (60) for retaining an intermediate portion of said cinch (42) along a central portion of said inner layer (32).

14. The menstrual garment of Claim 12 wherein said elongated piece of elastic material requires less than 800 grams of force to elongate 25%.

15. The menstrual garment of Claim 12 wherein said elongated piece of elastic material requires less than 400 grams of force to elongate 25%.

16. The menstrual garment of Claim 12 wherein said elongated piece of elastic material requires less than 200 grams of force to elongate 25%.

17. The menstrual garment of Claim 1 wherein said lifting means is a deformation member (200) which lifts said catamenial pad during use due to lateral compression forces of the female's thighs.

18. The menstrual garment of Claim 17 wherein said deformation member has a "C"-shaped cross-section (216) when worn.

19. The menstrual garment of Claim 17 wherein said deformation member has a "W"-shaped cross-section (220) when worn.

20. The menstrual garment of Claim 19 wherein said deformation member includes at least one compression line to facilitate bending parallel to a length of the deformation member.

21. The menstrual garment of Claim 17 wherein said deformation member is made of polyethylene film.

22. A menstrual short according to claim 1 wherein said lifting means is a stitched seam (112).

23. The menstrual short of Claim 22 wherein said stitched seam (112) has a front end and a rear end, said front end being located at least anterior to the anus of the female wearer.

24. The menstrual short of Claim 23 wherein said rear end extends at least 2.75" from a lowest portion of said outer layer when the menstrual short is folded along its first and second lateral sides.

25. The menstrual short of Claim 23 wherein said rear end of said stitched seam (112) exends to a position adjacent an elastic waistband of the menstrual short.

26. The menstrual short of Claim 23 wherein said rear end of said stitched seam (112) extends to a position at least above the curvature of the buttocks of the female wearer.

## Patentansprüche

1. Ein Menstruationskleidungsstück (28) zum Halten eines Monatshygienekissens (26) in einem Schambereich einer Trägerin, welches eine waschbare und wiederverwendbare Unterhose oder Short (28) umfaßt, welches umfaßt:
eine äußere Schichte 30;
erste (16) und zweite (18) Bein-Öffnungen, welche in der genannten äußeren Schichte (30) zum Aufnehmen von Beinen der Trägerin ausgebildet sind;
eine innere Schichte (32), welche zwischen den genannten ersten (16) und zweiten (18) Bein-Öffnungen angeordnet und an der genannten äußeren Schichte (30) fixiert ist, und wobei das genannte Menstruationskleidungsstück weiters ein Monatshygienekissen umfaßt, welches an der genannten äußeren Schichte (30) festgehalten ist, und wobei die genannte Short (28) umfaßt
ein Mittel zum Anheben, welches aus einem Gurtbauteil (42), einem Verformungsbauteil (200) oder einem gehefteten Saum (112) ausgewählt ist, dadurch gekennzeichnet, daß das genannte Mittel zum Anheben, um das genannte Monatshygienekissen (26), beim Tragen durch die Trägerin im Schambereich, in die Afterfurche und gegen das Perineum der Frau hochzuziehen, zwischen der genannten inneren Schichte (32) und der genannten äußeren Schichte (30) angeordnet ist.

2. Das Menstruationskleidungsstück nach Anspruch 1, welches weiters Befestigungsmittel zum Fixieren der genannten Mittel zum Anheben an einer der genannten inneren Schichte (32) und äußeren Schichte (30) umfaßt.

3. Das Menstruationskleidungsstück nach Anspruch 2, bei welchem das genannte Befestigungsmittel ein Haken- und Schlaufenbefestigungssystem ist.

4. Das Menstruationskleidungsstück nach Anspruch 2, bei welchem das genannte Befestigungsmittel ein Klebstoff ist.

5. Das Menstruationskleidungsstück nach Anspruch 1, bei welchem das genannte Mittel zum Anheben ein Gurtbauteil (42) ist.

6. Das Menstruationskleidungsstück nach Anspruch 5, bei welchem ein vorderes Ende des genannten Gurtes an mindestens einer der genannten inneren und äußeren Schichten an einer Stelle, welche vor dem Anus der Frau ist, befestigt ist.

7. Das Menstruationskleidungsstück nach Anspruch 5, bei welchem ein vorderes Ende des genannten Gurtes an mindestens einer der genannten inneren und äußeren Schichten an einer Stelle befestigt ist, welche benachbart zum Scheidenvorhof der Frau ist.

8. Das Menstruationskleidungsstück nach Anspruch 5, welches weiters Haltemittel zum Positionieren eines dazwischenliegenden Abschnitts der genannten Gurtvorrichtung entlang einem mittleren Längsabschnitt der genannten inneren Schichte inkludiert.

9. Das Menstruationskleidungsstück nach Anspruch 5, bei welchem ein hinteres Ende des genannten Gurtes an mindestens einer der genannten inneren und äußeren Schichten an einer Stelle befestigt ist, welche oberhalb der Krümmung der Backen der Frau ist.

10. Das Menstruationskleidungsstück nach Anspruch 5, bei welchem ein hinteres Ende des genannten Gurtes an mindestens einer der genannten inneren und äußeren Schichten an einer Stelle befestigt ist, welche benachbart einem elastischen Taillenband der genannten Menstruationshose ist.

11. Das Menstruationskleidungsstück nach Anspruch 5, bei welchem ein hinteres Ende des genannten Gurtes an mindestens einer der genannten inneren und äußeren Schichten in einer Position von mindestens 2,75" von einem niedrigsten Abschnitt der genannten äußeren Schichte, wenn die genannte Menstruationshose entlang ihren ersten und zweiten Längsseiten flach gefaltet ist, befestigt ist.

12. Das Menstruationskleidungsstück nach Anspruch 5, bei welchem der genannte Gurt (42) aus einem länglichen Stück elastischen Materials gebildet ist, mit einem ersten Ende (44) und einem zweiten Ende (48), wobei das genannte erste Ende (44) an einer der genannten inneren (32) und äußeren Schichten (30) an einer Stelle vor dem Anus der Trägerin fixiert ist, und das genannte zweite Ende (48) an einer der genannten inneren (32) und äußeren Schichten (30) an einer Stelle, welche den Backen der Trägerin benachbart ist, fixiert ist.

13. Das Menstruationskleidungsstück nach Anspruch 12, welches weiters Führungsmittel (60) zum Zurückhalten eines dazwischenliegenden Abschnitts des genannten Gurts (42) entlang einem mittleren Abschnitt der genannten inneren Schichte (32) umfaßt.

14. Das Menstruationskleidungsstück nach Anspruch 12, bei welchem das genannte längliche Stück aus elastischem Material weniger als 800 Gramm Kraft zur 25%-igen Verlängerung erfordert.

15. Das Menstruationskleidungsstück nach Anspruch 12, bei welchem das genannte längliche Stück aus elastischem Material weniger als 400 Gramm Kraft zur 25 %-igen Verlängerung erfordert.

16. Das Menstruationskleidungsstück nach Anspruch 12, bei welchem das genannte längliche Stück aus elastischem Material weniger als 200 Gramm Kraft zur 25 %-igen Verlängerung erfordert.

17. Das Menstruationskleidungsstück nach Anspruch 1, bei welchem das genannte Mittel zum Anheben ein Verformungsbauteil (200) ist, welcher das genannte Monatshygienekissen während des Gebrauchs zufolge von seitlichen Kompressionskräften der Schenkel der Frau anhebt.

18. Das Menstruationskleidungsstück nach Anspruch 17, bei welchem der genannte Verformungsbauteil beim Tragen einen 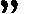 C"-förmigen Querschnitt (216) aufweist.

19. Das Menstruationskleidungsstück nach Anspruch 17, bei welchem der genannte Verformungsbauteil beim Tragen einen 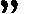 W"-förmigen Querschnitt (220) aufweist.

20. Das Menstruationskleidungsstück nach Anspruch 19, bei welchem der genannte Verformungsbauteil mindestens eine Kompressionslinie inkludiert, um ein Biegen parallel zu einer Länge des Verformungsbauteils zu ermöglichen.

21. Das Menstruationskleidungsstück nach Anspruch 17, bei welchem der genannte Verformungsbauteil aus Polyethylenfolie ist.

22. Eine Menstruationshose nach Anspruch 1, bei welcher das genannte Mittel zum Anheben ein gehefteter Saum (112) ist.

23. Die Menstruationshose nach Anspruch 22, bei welcher der genannte geheftete Saum (112) ein vorderes Ende und ein hinteres Ende aufweist, wobei das genannte vordere Ende mindestens vor dem Anus der Trägerin angeordnet ist.

24. Die Menstruationshose nach Anspruch 23, bei welcher das genannte hintere Ende sich mindestens 2,75" von einem niedrigsten Abschnitt der genannten äußeren Schichte erstreckt, wenn die Menstruationshose entlang ihren ersten und zweiten Längsseiten gefaltet ist.

25. Die Menstruationshose nach Anspruch 23, bei welcher das genannte hintere Ende des genannten gehefteten Saumes (112) sich zu einer Position, welche benachbart einem elastischen Taillenband der Menstruationshose ist, erstreckt.

26. Die Menstruationshose nach Anspruch 23, bei welcher das genannte hintere Ende des genannten gehefteten Saumes (112) sich zu einer Position mindestens oberhalb der Krümmung der Backen der Trägerin erstreckt.

## Revendications

1. Sous-vêtement périodique (28) destiné à maintenir un tampon cataménial (26) dans la région vulvaire d'une utilisatrice, comprenant une culotte ou un slip (28) lavable et réutilisable présentant :
une couche extérieure (30);
des première (16) et deuxième (18) ouvertures de jambes formées dans ladite couche extérieure (30), destinées à recevoir les jambes de l'utilisatrice:
une couche intérieure (32) disposée entre lesdites première (16) et deuxième (18) ouvertures de jambes et fixée à ladite couche extérieure (30), et ledit sous-vêtement périodique comportant, en outre, un tampon cataménial retenu sur ladite couche extérieure (30), et ledit slip (28) comprenant
un moyen de levage choisi entre un élément formant sangle (42), un élément de déformation (200) ou une couture (112), caractérisé en ce que ledit moyen de levage est situé entre ladite couche intérieure (32) et ladite couche extérieure (30) pour lever ledit tampon cataménial (26) quand il est porté par l'utilisatrice dans la région vulvaire, dans le sillon fessier et contre le périnée de la femme.

2. Sous-vêtement périodique selon la revendication 1, comprenant, en outre, des moyens de fixation pour assujettir ledit moyen de levage à l'une desdites couches intérieure (32) et extérieure (30).

3. Sous-vêtement périodique selon la revendication 2, dans lequel ledit moyen de fixation est un système de fixation à crochet et à boucle.

4. Sous-vêtement périodique selon la revendication 2, dans lequel ledit moyen de fixation est un adhésif.

5. Sous-vêtement périodique selon la revendication 1, dans lequel ledit moyen de levage est un élément formant sangle (42).

6. Sous-vêtement périodique selon la revendication 5, dans lequel une extrémité avant de ladite sangle est fixée à au moins une desdites couches intérieure et extérieure en un emplacement qui est antérieur à l'anus de la femme.

7. Sous-vêtement périodique selon la revendication 5, dans lequel une extrémité avant de ladite sangle est fixée à au moins une desdites couches intérieure et extérieure en un emplacement qui est adjacent au vestibule vaginal de la femme.

8. Sous-vêtement périodique selon la revendication 5, comprenant, en outre, un moyen de positionnement pour placer une partie intermédiaire dudit moyen formant sangle le long d'une partie longitudinale centrale de ladite couche intérieure.

9. Sous-vêtement périodique selon la revendication 5, dans lequel une extrémité arrière de ladite sangle est fixée à au moins une desdites couches intérieure et extérieure en un emplacement qui est au-dessus de la courbure des fesses de la femme.

10. Sous-vêtement périodique selon la revendication 5, dans lequel une extrémité arrière de ladite sangle est fixée à au moins une desdites couches intérieure et extérieure en un emplacement qui est adjacent à une ceinture élastique dudit slip périodique.

11. Sous-vêtement périodique selon la revendication 5, dans lequel une extrémité arrière de ladite sangle est fixée à au moins une desdites couches intérieure et extérieure, dans une position située au moins à 2,75" d'une partie la plus basse de ladite couche extérieure quand ledit slip périodique est plié à plat le long de ses premier et deuxième côtés latéraux.

12. Sous-vêtement périodique selon la revendication 5, dans lequel ladite sangle (42) est réalisée avec une pièce allongée de matériau élastique qui présente une première extrémité (44) et une deuxième extrémité (48), ladite première extrémité (44) étant assujettie à une desdites couches intérieure (32) et extérieure (30) en un emplacement antérieur à l'anus de l'utilisatrice, et ladite deuxième extrémité (48) étant assujettie à une desdites couches intérieure (32) et extérieure (30) en un emplacement qui est adjacent aux fesses de l'utilisatrice.

13. Sous-vêtement périodique selon la revendication 12, comprenant, en outre, un moyen de guidage (60) pour retenir une partie intermédiaire de ladite sangle (42) le long d'une partie centrale de ladite couche intérieure (32).

14. Sous-vêtement périodique selon la revendication 12, dans lequel ladite pièce allongée de matériau élastique nécessite une force inférieure à 800 grammes pour s'allonger de 25 %.

15. Sous-vêtement périodique selon la revendication 12, dans lequel ladite pièce allongée de matériau élastique nécessite une force inférieure à 400 grammes pour s'allonger de 25 %.

16. Sous-vêtement périodique selon la revendication 12, dans lequel ladite pièce allongée de matériau élastique nécessite une force inférieure à 200 grammes pour s'allonger de 25 %.

17. Sous-vêtement périodique selon la revendication 1, dans lequel ledit moyen de levage est un élément de déformation (200) qui soulève ledit tampon cataménial durant l'utilisation en raison des forces de compression latérales des cuisses de la femme.

18. Sous-vêtement périodique selon la revendication 17, dans lequel ledit élément de déformation a une section transversale (216) en forme de "C" quand il est porté.

19. Sous-vêtement périodique selon la revendication 17, dans lequel ledit élément de déformation a une section transversale (220) en forme de "W" quand il est porté.

20. Sous-vêtement périodique selon la revendication 19, dans lequel ledit élément de déformation comprend, au moins, une ligne de compression pour faciliter une flexion parallèle à une longueur de l'élément de déformation.

21. Sous-vêtement périodique selon la revendication 17, dans lequel ledit élément de déformation est réalisé avec un film de polyéthylène.

22. Slip périodique selon la revendication 1, dans lequel ledit moyen de levage est une couture (112).

23. Slip périodique selon la revendication 22, dans lequel ladite couture (112) présente une extrémité avant et une extrémité arrière, ladite extrémité avant étant située au moins de manière antérieure à l'anus de l'utilisatrice.

24. Slip périodique selon la revendication 23, dans lequel ladite extrémité arrière s'étend, au moins, de 2,75" d'une partie la plus basse de ladite couche extérieure quand le slip périodique est plié suivant ses premier et deuxième côtés latéraux.

25. Slip périodique selon la revendication 23, dans lequel ladite extrémité arrière de ladite couture (112) s'étend jusqu'à une position adjacente à une ceinture élastique du slip périodique.

26. Slip périodique selon la revendication 23, dans lequel ladite extrémité arrière de ladite couture (112) s'étend jusqu'à une position située au moins au-dessus de la courbure des fesses de l'utilisatrice.
